# EUROPEAN PATENT APPLICATION

(11) **EP 2 617 395 A1**
(43) Date of publication of application: **24.07.2013**
(21) Application number: 13152054.6
(22) Date of filing: 21.01.2013
(51) Int. Cl.: A61F 5/453, A61G 9/00

(54) **Urine collection device**

(30) Priority: 23.01.2012 DK 201270040
(71) Applicant: Urologic Aps, 8660 Skanderborg (DK)
(72) Inventor: Rødsten, Carsten Bob, 4070 Kirke Hyllinge (DK); Christensen, Thomas Olund, 2500 Valby (DK); Bender, Vagn, 6430 Nordborg (DK)
(74) Representative: Carlsson, Eva

(57) **Abstract**

A urine collection device (100) for a male user comprising: a receiver (1) for receiving urine comprising a distal end (13), a proximal end (12), a side wall (14) and an opening (15) having a size, a container (5) for collecting the urine; wherein the receiver (1) and the container (5) are adapted to be in liquid communication, wherein the proximal end of the receiver (1) has a circumference and the receiver (1) has a surrounding membrane section (19) positioned between the circumference and the opening (15), and that said receiver (1) is adapted to assume a receiving position and a rest position, such that the size of the opening (15) is adjustable between the receiving position and the rest position.

## Description

The present invention relates to medical devices, in particular to a urine collection device for male users, comprising: a receiver for receiving urine comprising a distal end, a proximal end, a side wall, an opening having a size and a container for collecting the urine; wherein the receiver and the container are adapted to be in liquid communication.

Various urinal devices have been developed for collecting urine from bedridden patients or from men who suffer from Lower Urinary Tract Symptoms (LUTS) and/or nocturia. By having to get up and attend to frequent toilet visits during the night they get their sleep interrupted. A way of avoiding the trip to the toilet is a bed side urinal. Typically, such devices include a urine receiving reservoir for holding and/or storing urine and a spout extending from the reservoir. The spout includes an inlet through which the urine is received.

A long recognized problem associated with portable male urinals, or bed bottles, is resisting backflow of the urine following use of the urinal or resisting the undesirable spillage of urine due to, for example, mishandling of the urinal. With this kind of urinal the user still have to more or less sit up in bed in order to avoid spillage and still have to be somewhat awake in order to avoid mishandling of the urinal. Another problem is that the nightly disturbance may also risk waking up the spouse, who also will get her sleep interrupted.

The above problems with these kinds of urinals have been attempted to be overcome for example as described in TW567843. Here a collection container for collecting the urine is provided with a soft tube connecting the collection container with a receiver. The soft tube is provided with a spring inside it, in order to make the tube stand in an upright position. The device is further provided with a back flow valve and a cover for the receiver. However this solution still requires that the user sits up in bed in order to avoid spillage as the receiver is not adapted sizewise to a particular user.

Other less relevant prior art examples can be found in CN102028593 and in CN2609539.

It is therefore the object of the invention to provide a device that eliminates spillage and is easy to use even when being sleepy.

This is achieved with a urine collection device wherein the proximal end of the receiver has a circumference and the receiver has a surrounding membrane section positioned between the circumference and the opening, and wherein said receiver is adapted to assume a receiving position and a rest position, such that a size of the opening is adjustable between the receiving position and the rest position. The membrane section is positioned between the circumference of the receiver and the opening in the rest position. When the receiver is in the receiving position the membrane section functions as a sealing membrane to prevent leakage when the membrane section is in the rest position. The rest position is assumed both when it is in use and when it is not in use. The receiving position is assumed when the receiver is positioned on the male member and when it is removed from the male member. The size is defined as the area within the circumference of or the perimeter of the opening.

In general, when using the term proximal and distal, by proximal is meant the end of a part that is closest to the user, and distal being the end that is usually closest to the floor or the bottom of the container, depending on the design of the urine collection device. For example the receiver is at the proximal end and the container is at the distal end. The receiver may be positioned directly on the container, for example where the container is in the form of a bed bottle, such that the user is able to lie down while using the device. Alternatively the receiver and the container are connected by means of a connector, such as a tube.

In the context of the present invention, the term "user" is meant to comprise the (male) person actually using (i.e. inserting the penis into) the receiver.

In the rest position the opening may be slightly smaller than the circumference of a penis shaft. In this way a snug fit is ensured, leaving no risk for spillage and is still comfortable for the user to use, as the opening is not too tight either. The penis shaft is typically between 20 and 30 mm in diameter, but it may be smaller or bigger. The opening in the rest position is preferably between 10 and 40 mm in diameter, more preferably between 15 and 35 mm in diameter, and most preferably between 20 and 30 mm in diameter.

The membrane section may be retractable, such that a part of the membrane section becomes part of the side wall in the receiving position. Thereby, no added parts are required, such as a lid, for at least partly closing of the receiver as the membrane section and the wall of the receiver is in one, which makes it easier to handle in the dark. The membrane section is adapted to slide over the edge constituting the circumference of the proximal end of the receiver and becoming a part of the side wall. The size of the opening is adjustable by pressing on the side wall of the receiver, which results in the membrane section sliding over the edge. Thereby, the opening, in the receiving position, is adapted to receive a penis that may easily enter the opening.

The receiver may comprise a cover and a flexible or resilient member, preferably comprising at least two legs, extending along the longitudinal axis. The legs may be fixed at the distal end of the receiver for example on a ring or a tube shaped element adapted to be connected with the connector for leading the urine into the container or the container. This construction makes the receiver resilient, such that it may give in to pressure when the two legs are pressed towards each other, and consequently adjusting the opening in the membrane section as the membrane section may slide over the edge of the receiver.

The member may be a flexible member comprising more legs or be of a different configuration. For example the resilient member may comprise two, three or four legs mounted on a ring, around which they are able to pivot. The ring may be positioned on a middle part of the legs, at a distance from the ends. The legs are angled around the ring, such that the distance between two legs at the distal end and the proximal end is greater than in the middle part where the legs are mounted on the ring. The legs are preferably flat and rounded at the proximal end, such that they follow the circumference of the receiver. The flexible member in this configuration is positioned inside the cover. The cover is preferably elastic. The opening in the receiver is then adjusted by applying a circumferential pressure on the legs at their distal end on the outside of the cover. This may be done by hand or by using a clamming device.

The receiver may further comprise a ring member provided inside the receiver immediately below the membrane section. The ring member keeps the circumference of the proximal end of the receiver constant, and the membrane section may slide over the ring member such that the opening in the receiver is made bigger in order to receive the male member.

Additionally the receiver may be provided with at least two substantially opposing pressure points provided below the ring member, which may be indicated on the cover or on the resilient or flexible legs as the legs may be provided inside or outside the cover. The legs may be made for example in an elastomer or polymer material.

In order for the ring member to be able let the membrane section retract and slide over the edge of the ring member more easily, the ring member may comprise a rigid ring enclosed in a toroidal shaped tube. The toroidal shaped tube may be corrugated. Alternatively or additionally the ring member may have a low friction coefficient, such as less than 0.5, such that the resistance between the cover and the ring member is reduced and the receiver is easier to use. In case the toroidal shaped tube is corrugated, it may be rotatable in the poloidal direction, in which case friction is not an issue. The term "poloidal direction", means the short way around the torus.

The cover preferably comprises a pliable material such as silicone or polyurethane. The membrane section may have a thickness of 0.2-0.4 mm, and/or the side wall of the receiver may have a thickness of 1-4 mm. This configuration has been found suitable such that the receiver is easier to manipulate into the desired position, but still being strong enough to withstand daily use.

The liquid communication may be established by means of a connector, which in a rest position preferably in a substantially upright position. The connector may be used both for a bed bottle and for a urine collection device adapted to be positioned on the floor. In a further embodiment the connector may be manufactured as a part of the receiver, such that the receiver and connector are in one piece.

The connector makes it possible to have a distance between the receiver and the container, such that the container may be positioned on the floor.

The upright position makes it easier for the user to reach out for the receiver that may be positioned in a height of approximately 60-85 cm, more preferably 70-80 cm above the floor, such that the receiver is approx 10-15 cm above the sleeping level.

The upright position may be achived with a tube with a shore A hardness of 70-90 used for the connector, the connector having a rigidity corresponding to that of a tube made of vulcanized rubber, silicone, thermoplastic elastomer (TPE) or similar with a material thickness of 2-6 mm, preferably 3-5 mm and an outer diameter between 15-35 mm. Instead of using only a tube as the connector, an intermediate piece may be used in addition to the connector, for example positioned between the connector and the container. The intermediate piece may be in a rigid or semi rigid material. As follows the tube may be more flexible as it still possible to obtain an upright position as well as a suitable height of the receiver. The tube or connector may be manufactured by extrusion or by molding.

As an alternative the connector may comprise a connector tube supported by a spring along the length of the connector tube, inside the tube lumen, interarted in the tube wall or outside the connector tube. This will likewise enable the receiver to stand in an upright position.

The container may comprise a bottom and an opening adapted to be connected with the connector, and an inclined surface provided inside the container, wherein the opening is positioned above the inclined surface, such that the urine is able to run down the inclined surface before reaching the bottom of the container. This makes the device more quit as the splashing sounds are substantially reduced.

The container is preferably in the form of a pitcher or a jug, which may be removable from the urine collection device or a housing. The pitcher shape makes it easier to empty the container after use. The container may also be a bed bottle.

In the following the invention will be described in relation to the drawings in which:
Fig 1 is a perspective view of an embodiment of the device according to the invention,
Fig. 2 is a perspective view of an embodiment of a container,
Fig. 3 is a perspective view of an embodiment of a container seen from above,
Figs 4a and 4b are schematic drawings of an embodiment of the receiver in the rest and the receiving position, respectively, seen from the side and from above,
Figs 5a and 5b are views of a second embodiment of the receiver and a cross section of the receiver, respectively,
Fig. 6 is a perspective view of a toroidal shaped tube,
Fig. 7 is a perspective view of a rigid ring,
Fig. 8 is a view of an embodiment of a resilient member and the cover,
Fig. 9 is a second embodiment of a flexible member, and
Fig. 10 is an embodiment of a clamming device.

Like reference numbers will be used for similar features throughout the application.

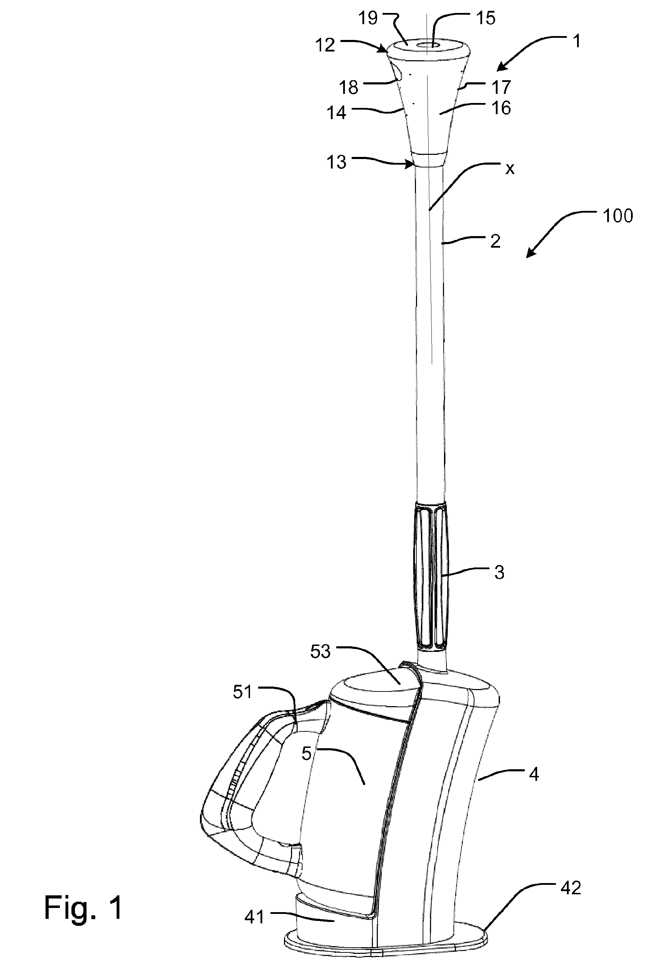

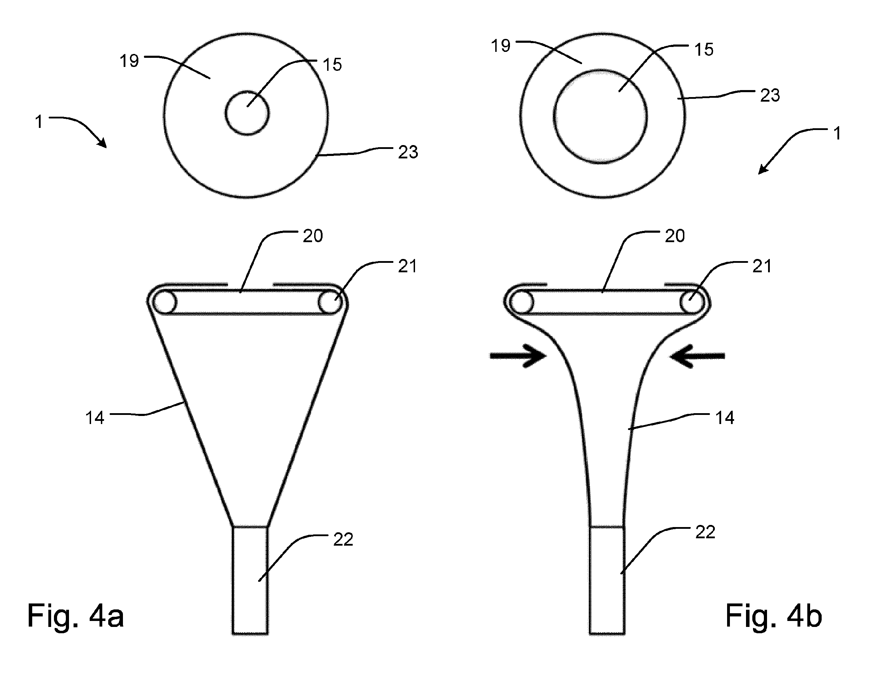

Fig. 1 shows an embodiment a urine collection device of the invention in a rest position. The urine collection device generally designated 100 comprises a funnel-shaped receiver 1 mounted on a connector 2. The connector 2 and the receiver 1 may be made in one piece. The receiver 1 may have other shapes, such as bowl-shaped. The receiver 1 comprises a cover 16 made of a pliable material, such as silicone, polymer or rubber. The receiver 1 further comprises an opening 15 at a proximal end 12 of the receiver 1. The opening 15 is defined in a membrane section 19 that extends between a side wall 14 of the receiver 1 and the opening 15. The membrane section 19 has the form of a brim. A longitudinal axis x positioned through the centre of opening 15 is defined. The opening 15 is substantially facing upwards, when not in use, and is adapted to receive the male member of a user. The receiver 1 further comprises a resilient member 17 provided on the outside of the cover 16. The resilient member 17 is used for supporting the cover 16. In the embodiment shown, the resilient member 17 is provided with two legs extending along the outside of the side wall 14 of the cover 16 and is attached at a distal end 13 of the receiver 1 opposite the proximal end 12. Each leg includes a pressure point 18 provided at the proximal end of the leg. More legs may be provided. The functionality of the receiver 1 will be described in further detail in relation to Figs 4-6.

The connector 2 is used for establishing a liquid connection between the receiver 1 and the container 5. The connector 2 is, in the embodimen shown, in the form of a tube in vulcanized rubber or alternatively silicone. The connector 2 is both flexible enough to be able to bend in an approximately 90 degrees angle, or between 70-90 degrees from the distal end to the proximal end of the connector 2, while at the same time being rigid enough to stand in an upright position carrying the receiver 1, when standing on a substantially plane surface, such as a floor. The upright position as shown makes it easier for the user, when the device is positioned next to the bed, to reach out for the receiver 1, which advantageously is positioned in a height of approx. 75 cm above the floor, which in many cases is a suitable height in relation the height of the bed. The desired flexibility of the connector 2 may be reached with a suitable material such as vulcanized rubber having a shore A hardness of 70-90, preferably 80-90, an outer diameter between 15-35 mm, preferably approx. 25 mm and a thickness of the wall in the connector of 2-6 mm, preferably approx. 3-5 mm. The height of the connector is approx. 30 cm or it may be shorter or longer. Alternatively a softer more flexible tube, in e.g. plastic, may be used, supported by for example a metal spring provided around the tube or inside the tube, where the spring makes it able to stand in an upright position, while maintaining its flexible properties. The spring may alternatively be made of polymers such as fiber reinforced polyester or epoxy. By making the connector 2 semi-flexible the user is able to pull the receiver 1 over the edge of the bed, and position his male member, i.e. penis, in the receiver 1 through the opening 15 while lying in the bed. The functionality of the receiver 1, which will be described later, will make it possible for him to void in the receiver while lying down without the risk of spilling any urine in the bed or elsewhere. This way the frequent nightly visits to the toilet can be exchanged with a minor disturbance that can be performed without getting out of bed and without turning on the light.

Below the connector 2 is an intermediate piece 3 with the purpose of extending the distance between the receiver 1 and the container 5. The intermediate piece 3 is preferably a rigid tube. By using an intermediate piece 3 a more flexible connector may be used as the connector is shortened on account of the intermediate piece 3. The intermediate piece 3 may be dispensed with or it may be provided separately, such that it is interchangeable and may be provided in different lengths for different heights of beds.

The intermediate piece 3, or the connector 2, is attached to the housing 4.

The connector 2 and the intermediate piece 3 may be dispensed with and instead the receiver 1 may be attached directly to the container 5. The container 5 is in this case preferably a bed bottle (not shown).

The housing 4 is provided with a base plate 42 to increase its stability. Additionally a wedge shaped platform 42 is used for receiving the container 5. The wedge shape makes it easier for the user to remove the container 5 from the housing 4 after use, as the device will often be positioned on the floor and the user will have to reach down to withdraw it from the housing 4. The housing further comprises a space for partly accommodating the container 5 as shown.

The device 1 further comprises a container 5 in the form of a pitcher or jug. The container 5 is provided with a handle 51 such that the container is easy to withdraw from the housing 4, and a lid 53 for easy access though the top when cleaning the container 5.

In order to avoid any odours from the container 5 a closing mechanism or a one way valve may be provided somewhere between the container 5 and the receiver 1.

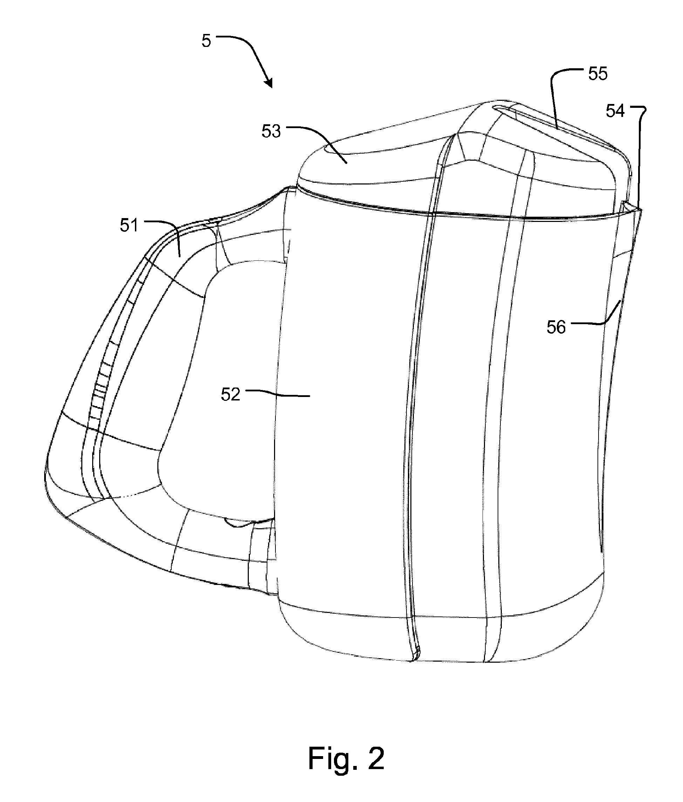

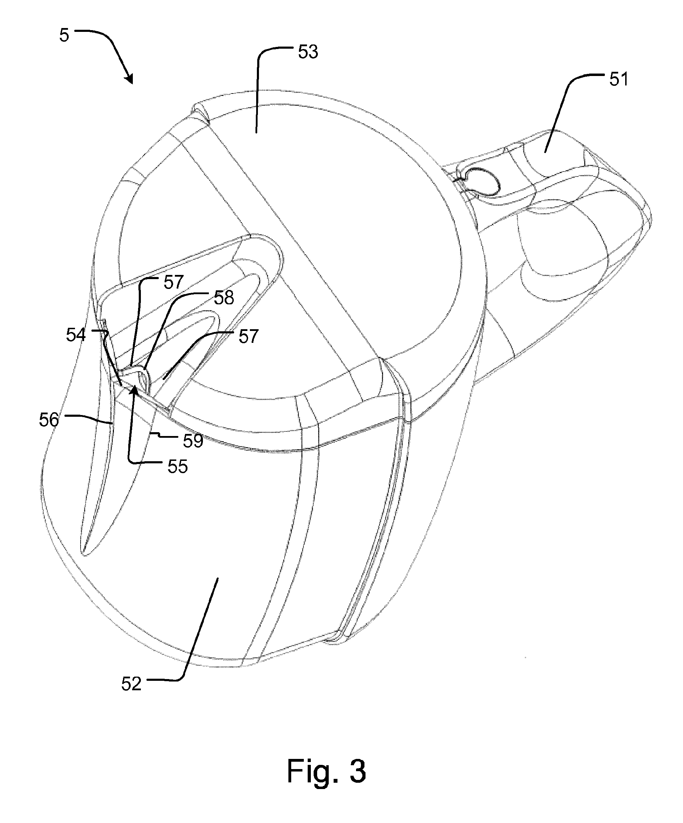

The container 5 itself is shown in Figs 2 and 3. Here it is shown that the container 5 further comprises a spout 54 for pouring the content out after use. The spout 54 is provided with a drip catcher, meaning that the urine is prevented from running down the outer side of the container 5 after the content has been poured out. This is facilitated by a thin edge of the spout 54. When the device is in use and urine is entering the container 5, the incurved shape of the container 56 below the spout 54 prevents the urine from hitting the bottom right away and making splashing sounds. Instead the urine will be running down the incurved shape of the container 56 below the spout 54, which will considerably reduce the splashing. As can be seen in Fig.3, two inclined surfaces 57 are provided in the lid 53 or in a fixed top for the urine to run down the surface 57 and in this way being led to the incurved shape of the container 56 below the spout 54, to address the splashing problem. The inclined surfaces 57 lead to the substantially vertical edges 59 of the internal wall of the spout as the distance from the lid 53 to the internal wall of the spout is shorter here than at the tip of the spout 54. So directly opposite the tip of the spout the lid 53 is curving upwards in a crease 58. The container 5 further comprises a hole 55 in the lid 53 or in a fixed part of the top for receiving the urine coming from the connector 2 or the intermediate piece 3 (see Fig. 1). The hole 55 is positioned between the inclined surfaces 57, the crease 58 and the internal wall of the container 56.

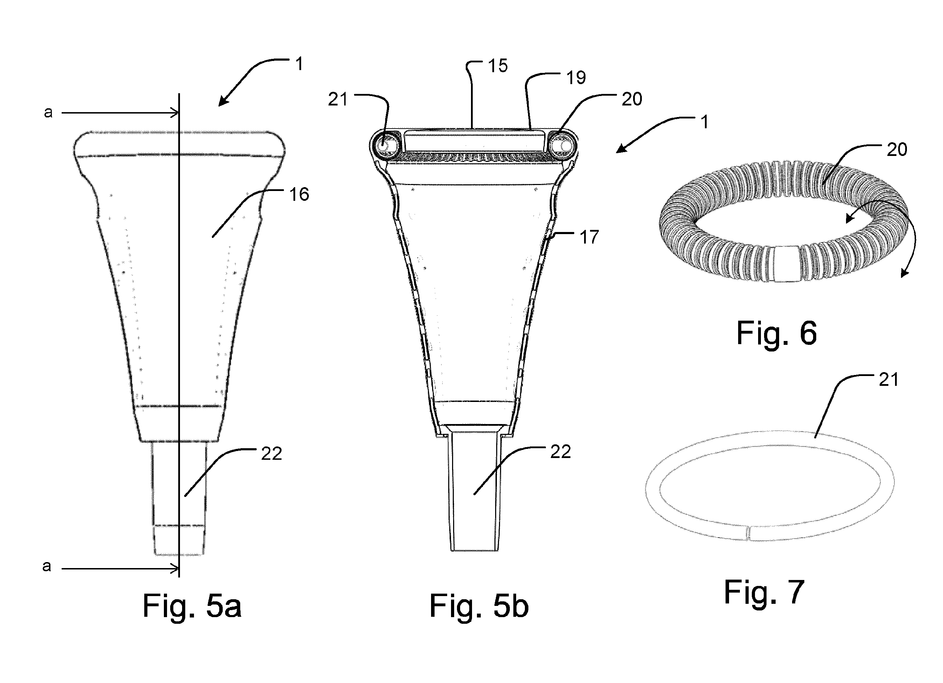

In Figs 4a and 4b the principle of function is shown. For further details in the drawing see Figs 5-7. In Fig.4a the receiver 1 is seen in a rest position from above and a cross section of the receiver 1, respectively. Here the side wall 14 is substantially straight and the opening 15 in the membrane section 19 is small. Thus, the opening 15 is made to be slightly smaller than the circumference of a penis shaft. As a penis shaft is typically between 20 and 30 mm in diameter (but may be smaller or bigger), and thus the diameter of the opening 15 typically lies in the same range 20 to 30 mm but slightly smaller. The device may be provided in different sizes, or it might be possible to adjust the size of the opening 15 after supply to the consumer. In this case, a very small opening of for instance 5 mm may be provided, which is then cut to the appropriate size. Thus in some cases, the opening is only approximately 5-20 mm in diameter. In Fig. 4b the side wall 14 has been pressed from two sides which results in the size of the opening 15 is getting larger. In both Figures, the outer circumference is kept the same size due to a rigid ring 21 provided inside the cover 16 of the receiver 1 below the membrane section 19. The rigid ring 21 is enclosed in a tube 20. What happens when the side wall 14 is pressed, the membrane section 19 moves over the circumference of the receiver 1 and a part of the membrane section 19 becomes a part of the side wall 14, thereby making the size of opening 15 bigger. The size of the opening 15 in the receiving position should thus be large enough to accommodate the shaft of a penis. As the penis is normally flaccid, it may occur, however, that it is slightly erect, and the dimensioning of the size of the opening 15 in the receiving position should thus be able to take this into account. Thus, it may thus be necessary to facilitate a diameter of the opening 15 in the receiving position of 40 mm or more. Furthermore, the receiver 1 should have such dimensions that it may accommodate a sufficiently large portion of the penis in the longitudinal direction to allow safe retention during urination.

In order for the membrane section 19 to retract, the tube 20 is corrugated as can be seen in Fig. 6. This enables the tube 20 to rotate in a poloidal direction due to the friction between the tube 20 and the cover 16. The ring 21 inside the tube does not rotate with the tube 20. Instead of a corrugated tube, a silicone tube with a low friction coefficient, such as less than 0.5, may be used. So instead of the tube 20 is rotating it is sliding against the cover 16. A lubricant may be helpful in facilitating the sliding.

In Fig. 5b a cross section of the receiver 1 is shown, which cross section is indicated by the vertical line a-a in Fig. 5a. In this embodiment the resilient member 17 has been positioned inside the cover 16. The receiver 1 further comprises a connecting piece 22 for joining the receiver 1 and the connector 2 by insertion of the connecting piece 22 into the connector 2.

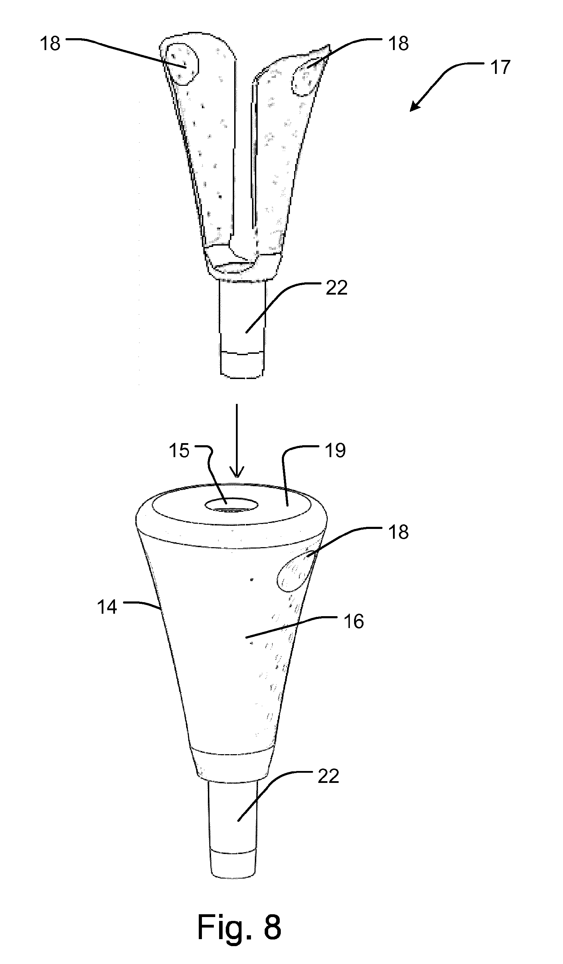

In Fig. 8 the resilient member 17 and the cover 16 can be seen separately. Here the resilient member 17 is adapted to go inside the cover 16. But the resilient member 17 may also be provided on the outside of the cover 16 as can be seen in Fig. 1. Both the flexible member 17 and the cover 16 are provided with pressure points 18 that in a mounted state overlap each other, respectively. So by pressing on the pressure points 18 the size of opening 15 in the membrane section 19 gets bigger and the male member may enter. When in use the receiver 1 is brought to its rest position by releasing the pressure points 18 and the opening 15 provides a snug fit around the male member. Thereby spillage is avoided. The elasticity of the membrane section 19 makes it comfortable to wear as well. When the user is done he presses on the pressure points 18 and the opening 15 gets bigger and he can retract his male member. When releasing the receiver 1 the size of opening 15 is reduced and the receiver 1 and the connector 2 are adapted to return to a substantially upright position. The membrane section 19 is made of a material with a thickness of 0.2-0.4 mm, which has proven to provide sufficient strength but still feel comfortable against the shaft of the penis.

The flexible member 17 may have more legs and/or pressure points around the circumference of the receiver 1.

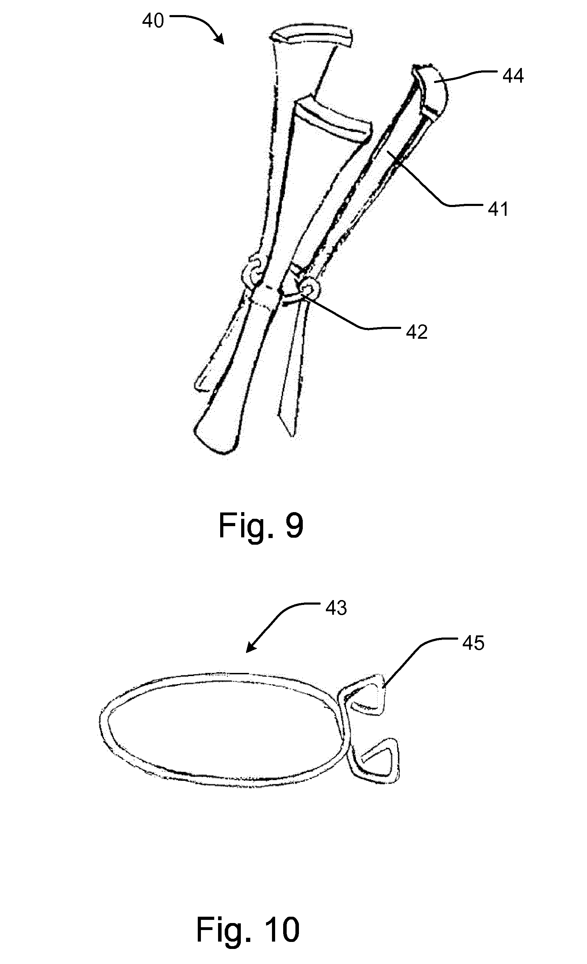

In Fig. 9 a further embodiment of the flexible member 40 is shown. This embodiment may replace the use of the embodiment of the resilient member as shown in the previous Figures. The flexible member 40 is adapted to be positioned inside the cover. The flexible member 40 comprises three legs, each provided with a lip 44 at the proximal end. The end is adapted to be positioned along the circumference of the proximal end inside the cover (not shown). The legs 41 are mounted on a ring 42. The ring 42 is mounted in a hole through each of the legs 41, which hole is positioned approximately in a middle part of the legs, between the distal and the proximal end. The legs 41 are able to pivot around the ring 42 in the poloidal direction. The legs 41 are bent such that the legs 41 are extending in diverging directions from the point of attachment to the ring 42, such that the flexible member 40 has a substantially hourglass shape. The cover may have a corresponding shape.

When circumferential pressure is applied around the legs 41 at their distal end, the legs 41 pivot around the ring 42 and the legs will be spread apart at the proximal end. As the cover is provided on the outside of the flexible member 40, this results in the circumference of the receiver being enlarged, and thereby the size of the opening in the section membrane as previously shown will also become bigger. Thereby it is easy to let a male member enter the opening. After the circumferential pressure on the legs is released, the receiver will assume the rest position, where the size of the opening is reduced again.

For creating the circumferential pressure a clamming device 43 as shown in Fig.10 may be used. This is positioned outside the cover over the distal end of the legs. The circumferential pressure on the legs is applied by pressing the two handles 45 together. This will decrease the circumference of the clamming device 43 and thereby applying a circumferential pressure of the legs 41 of the flexible member 40.

In general, the features of the embodiments shown and described may be combined freely and no feature should be seen as essential unless stated in the independent claim.

## Claims

1. A urine collection device (100) for a male user comprising:
a receiver (1) for receiving urine comprising a distal end (13), a proximal end (12), a side wall (14) and an opening (15) having a size,
a container (5) for collecting the urine;
wherein the receiver (1) and the container (5) are adapted to be in liquid communication; **characterized in that** the proximal end of the receiver (1) has a circumference and the receiver (1) has a surrounding membrane section (19) positioned between the circumference and the opening (15), and that said receiver (1) is adapted to assume a receiving position and a rest position, such that the size of the opening (15) is adjustable between the receiving position and the rest position.

2. A urine collection device (100) according to claim 1, wherein in the rest position the opening (15) is slightly smaller than the circumference of a penis shaft.

3. A urine collection device (100) according to claim 1 or 2, wherein the membrane section (19) is retractable, such that a part of the membrane section (19) becomes part of the side wall (14) in the receiving position.

4. A urine collection device (100) according to any one of the preceding claims, wherein the receiver (1) comprises a cover (16) and a flexible or resilient member (17; 40), preferably comprising at least two legs, extending along a longitudinal axis (x).

5. A urine collection device (100) according to any one of the preceding claims, further comprising a ring member (20, 21) provided inside the receiver (1) immediately below the membrane section (19).

6. A urine collection device (100) according to claim 5, wherein the receiver (1) is provided with at least two substantially opposing pressure points provided below the ring member (20, 21).

7. A urine collection device (100) according to claim 5, wherein the ring member (20, 21) comprises a rigid ring (20) enclosed in a toroidal shaped tube (21).

8. A urine collection device (100) according to claim 7, wherein the toroidal shaped tube (21) is corrugated.

9. A urine collection device (100) according to claim 7 or 8, wherein the toroidal shaped tube (21) is rotatable in a poloidal direction.

10. A urine collection device (100) according to claim 4, wherein the cover (16) comprises a pliable material such as silicone or polyurethane.

11. A urine collection device (100) according to any one of the preceding claims, wherein the membrane section (19) is made of a material with a thickness of 0.2-0.4 mm and/or the side wall (14) is made of a material with a thickness of 1-3 mm.

12. A urine collection device (100) according to any one of the preceding claims, wherein the liquid communication is established by means of a connector (2), which in the rest position preferably is in a substantially upright position.

13. A urine collection device (100) according to claim 12, wherein the connector (2) has a rigidity corresponding to that of a tube made of vulcanized rubber or silicone with a Shore A hardness of 70-90, with a material thickness of 2-6 mm and an outer diameter between 15-35 mm.

14. A urine collection device (100) according to claim 12 or 13, wherein the connector (2) comprises a connector tube supported by a spring along the length of the tube.

15. A urine collection device (100) according to any one of the preceding claims, wherein the container (5) is a pitcher or a jug, which is removable from the urine collection device (100).
